# EUROPEAN PATENT APPLICATION

(11) **EP 4 596 701 A1**
(43) Date of publication of application: **06.08.2025**
(21) Application number: 23872402.5
(22) Date of filing: 27.09.2023
(51) Int. Cl.: C12N 15/87, C07K 17/02, C12N 15/09

(54) **CARRIER FOR PROTEIN, AND PROTEIN INTRODUCTION METHOD**

(30) Priority: 29.09.2022 JP 2022156126
(71) Applicant: TOKYO MEDICAL UNIVERSITY, Tokyo 160-8402 (JP)
(72) Inventor: KURODA Masahiko, Tokyo 160-8402 (JP); UMEZU Tomohiro, Tokyo 160-8402 (JP)
(74) Representative: Dehns
(86) International application number: PCT/JP2023/035106
(87) International publication number: WO 2024/071180

(57) **Abstract**

Provided is a novel carrier for introducing a protein such as a Cas protein into cells. The carrier for a protein according to the present invention includes vesicles of a fruit of a plant in the family Malpighiaceae, and a substance to be held is a protein or a nucleic acid encoding the protein. The plant in the family Malpighiaceae is, for example, an acerola plant species (Malpighia sp.). The carrier for a protein according to the present invention can be used as, for example, a tool for genome editing.

## Description

### Technical Field

The present invention relates to a carrier for a protein and a protein introduction method.

### Background Art

In the field of biochemistry, great importance is placed on DDS (drug delivery system) platforms for delivering a target substance to a target cell.

For example, in genome editing, which is widely used in various fields such as agriculture, livestock farming, fisheries, and medicine, the Cas9 protein with nuclease activity and a guide RNA, which is an RNA molecule, are brought into contact with a target sequence to cleave the target sequence, thereby knocking out a gene or knocking in a foreign gene. Thus, genome editing requires, for example, the delivery of proteins with relatively large molecular weights. Furthermore, when it comes to introducing proteins into animals and plants for food, or into humans for medical treatment, there are concerns about effects of artificial DDS on a living organism, for example.

### Summary of the Invention

### Technical Problem

In view of this, an object of the present invention is to provide a novel carrier, which can serve as a DDS platform for proteins.

### Solution to the Problem

In order to achieve the above object, a carrier for a protein according to the present invention includes vesicles of a fruit of a plant in the family Malpighiaceae, and a substance to be held is a protein or a nucleic acid encoding the protein.

A method for producing a protein delivery reagent according to the present invention includes allowing the carrier for a protein including the vesicles of the present invention and a protein to be delivered or a nucleic acid encoding the protein to coexist in a solvent to thereby form a conjugate of the vesicles of the carrier for a protein and the protein or the nucleic acid encoding the protein.

A method for introducing a protein into cells according to the present invention includes bringing a conjugate of the carrier for a protein including the vesicles of the present invention and a protein to be delivered or a nucleic acid encoding the protein into contact with cells.

### Advantageous Effects of Invention

The carrier for a protein according to the present invention includes vesicles of a fruit of a plant in the family Malpighiaceae, such as acerolas, and can hold a protein or a nucleic acid encoding the protein by the vesicles. Therefore, the carrier for a protein according to the present invention can be used, for example, as a DDS tool for proteins, such as nucleases used in genome editing or the like.

### Brief Description of Drawings

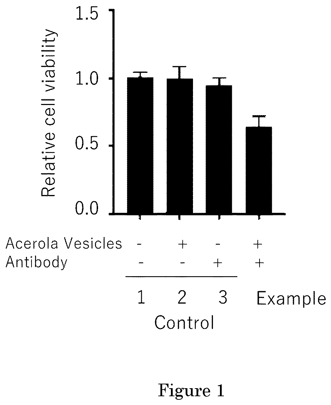

Figure 1 is a graph showing the cell viability of cells into which an anti-mTOR antibody was introduced using the carrier for a protein according to the present invention in Working Example 3.

### Description of Embodiments

Unless otherwise specified, the terms used herein may be used in the sense commonly used in the art.
[1] A carrier for a protein, comprising vesicles of a fruit of a plant in the family Malpighiaceae, wherein a substance to be held is a protein or a nucleic acid encoding the protein.
[2] The carrier for a protein according to [1], wherein the plant in the family Malpighiaceae is an acerola plant species (Malpighia sp.).
[3] The carrier for a protein according to [1] or [2], wherein the vesicles have an average particle size of 30 to 400 nm.
[4] The carrier for a protein according to any one of [1] to [3], wherein the vesicles are extracellular vesicles.
[5] The carrier for a protein according to any one of [1] to [4], wherein the nucleic acid encoding the protein is an expression vector in which a coding sequence for the protein is inserted into a vector.
[6] The carrier for a protein according to any one of [1] to [5], wherein the protein is a sequence-specific nuclease.
[7] The carrier for a protein according to [6], wherein the sequence-specific nuclease is a Cas protein.
[8] The carrier for a protein according to [7], wherein the Cas protein is a Cas9 protein.
[9] The carrier for a protein according to any one of [1] to [8], wherein the substance to be held further contains a concomitant component for the protein or the nucleic acid encoding the protein.
[10] The carrier for a protein according to [9], wherein the concomitant component is a nucleic acid substance, and the nucleic acid substance is a guide RNA.
[11] The carrier for a protein according to any one of [1] to [10], further including the protein or the nucleic acid encoding the protein.
[12] A method for producing a protein delivery reagent, including allowing the carrier for a protein including the vesicles according to any one of [1] to [10] and a protein to be delivered or a nucleic acid encoding the protein to coexist in a solvent to thereby form a conjugate of the vesicles of the carrier for a protein and the protein or the nucleic acid encoding the protein.
[13] The method for producing a protein delivery reagent according to [12], including mixing and incubating the carrier for a protein and the protein or the nucleic acid encoding the protein in the solvent.
[14] The method for producing a protein delivery reagent according to [12] or [13], further including allowing a concomitant component for the protein or the nucleic acid encoding the protein to coexist in the solvent together with the carrier for a protein.
[15] A method for introducing a protein into cells, including bringing a conjugate of the carrier for a protein including the vesicles according to any one of [1] to [10] and a protein to be delivered or a nucleic acid encoding the protein into contact with cells.
[16] The protein introduction method according to [15], wherein the conjugate is a conjugate further containing a concomitant component for the protein or the nucleic acid encoding the protein.
[17] The protein introduction method according to [15] or [16], further including, prior to the bringing of the conjugate into contact with the cells, forming a conjugate of the carrier for a protein and the protein or the nucleic acid encoding the protein.
[18] The protein introduction method according to any one of [15] to [17], wherein the bringing of the conjugate into contact with the cells is bringing the conjugate into contact with cells in vivo, ex vivo, or in vitro.
[19] The protein introduction method according to any one of [15] to [18], wherein the bringing of the conjugate into contact with the cells is administering the conjugate to a human or a non-human animal.
[20] A protein delivery reagent including the carrier for a protein according to any one of [1] to [10], and a protein or a nucleic acid encoding the protein.
[21] The protein delivery reagent according to [20], wherein the nucleic acid encoding the protein is an expression vector in which a coding sequence for the protein is inserted into a vector.
[22] The protein delivery reagent according to [20] or [21], wherein the protein is a sequence-specific nuclease.
[23] The protein delivery reagent according to [22], wherein the sequence-specific nuclease is a Cas protein.
[24] The protein delivery reagent according to [23], wherein the Cas protein is a Cas9 protein.
[25] The protein delivery reagent according to [20] to [24], further including a concomitant component for the protein or the nucleic acid encoding the protein.
[26] The protein delivery reagent according to [25], wherein the concomitant component is a nucleic acid substance, and the nucleic acid substance is a guide RNA.

### (1) Carrier for Protein

As described above, a carrier for a protein (a protein carrier) of the present invention includes vesicles of a fruit of a plant in the family Malpighiaceae. The inventor of the present invention found that vesicles of a fruit of a plant in the family Malpighiaceae hold a protein or a nucleic acid encoding the protein to form a conjugate, the conjugate is taken up into cells (also referred to as "target cells" hereinafter), and the protein or a protein expressed from the protein-coding nucleic acid can function in the cells, and thus established the present invention. The feature of the present invention is that the protein carrier includes the vesicles, and there are no particular limitations on the other configurations and conditions.

As described above, the protein carrier of the present invention can hold the protein or the nucleic acid encoding the protein by the vesicles, and by holding the protein or the nucleic acid encoding the protein, the protein carrier can deliver the protein or the nucleic acid encoding the protein. Therefore, the protein carrier of the present invention is also called, for example, a delivery reagent or a DDS reagent. In the present invention, a "protein carrier" refers to, for example, a carrier that holds a protein itself, as well as a carrier that holds a nucleic acid encoding a protein. When the protein-coding nucleic acid that is held by the protein carrier is, for example, introduced into cells by the protein carrier, the encoded protein can be synthesized by translational machinery for translation into the protein in the cells. Thus, it is sufficient that the protein-coding nucleic acid includes, for example, a sequence (e.g., ORF) that serves as a reading frame that enables a protein to be synthesized by the translational machinery of the cells. When holding the protein-coding sequence, the protein carrier of the present invention can also be referred to as, for example, a carrier for a protein-coding nucleic acid.

There is no particular limitation on the type of plant in the family Malpighiaceae, and examples thereof include plants of the genus Malpighia, more specifically, for example, acerola species (Malpighia sp.) and the like, and preferably acerolas such as M. emarginata DC., M. glabra, and M. punicifolia.

The vesicles can be obtained from the fruit of the plant in the family Malpighiaceae. The fruit may be fully ripe or unripe (not yet be fully ripe), or may be a mixture thereof, for example. It is preferable that the vesicles are, for example, in the form of a vesicular fraction that is collected from the juice of the fruit and that will be described later.

The vesicles can be prepared by, for example, extraction from the above-described fruit of the plant in the family Malpighiaceae. There is no particular limitation on the preparation method, and, for example, the vesicles can be obtained by crushing the fruit, preparing a crushed product or a suspension of the crushed product, and fractionating the vesicles. There is no particular limitation on the fractionating method, and examples thereof include an ultrafiltration method, an ultracentrifugation method, a concentration gradient method, a separation method using a micro liquid system, or other methods. The preparation method may be performed using, for example, a commercially available kit, and ExoEasy Maxi Kit (trade name, available from QIAGEN), ExoQuick (trade name, available from System Bioscience), a Total Exosome Isolation reagent (trade name, available from Invitrogen), and the like can be used. The vesicles may also be prepared by, for example, obtaining a juice by squeezing the fruit, and subjecting the thus obtained juice to any of various separation methods such as those described above. For example, a grinding device, a squeezing device, or the like can be used to prepare the juice. The juice may be, for example, any of a juice obtained from a fully ripe fruit, a juice obtained from an unripe fruit, and a juice obtained from a frozen fruit that may be fully ripe or unripe.

For example, a vesicular fraction containing a plurality of vesicles described above can be used for the protein carrier of the present invention. There is no particular limitation on the size of the vesicles, and the vesicles can have a particle size of, for example, 30 to 400 nm, 80 to 300 nm, 150 to 300 nm, 150 to 250 nm, 200 to 250 nm, 100 to 200 nm, or 80 to 200 nm. The vesicles may also be referred to as, for example microvesicles or nanovesicles. When expressed the particle size using a particle size distribution, a peak particle size of the vesicular fraction is not particularly limited, and is, for example, 30 to 400 nm, 80 to 300 nm, 150 to 300 nm, 150 to 250 nm, 200 to 250 nm, 100 to 200 nm, 80 to 200 nm, 200 ± 100 nm, 200 ± 50 nm, 200 ± 30 nm, or 200 ± 20 nm. Moreover, in the particle size distribution, when a total of the vesicles is taken as 100%, a percentage of vesicles at the above-described peak (e.g., 200 ± 50 nm, 200 ± 20 nm) is not particularly limited, and the lower limit is, for example, 30% or more, 50% or more, or 80% or more, and the upper limit is, for example, 70% or less, 80% or less, 90% or less, or 100%. The vesicular fraction is, for example, a fraction extracted from a juice of the fruit so as to have the above-described particle size and particle size distribution. In the case where the vesicular fraction is used for the protein carrier of the present invention, for example, a fraction that has been extracted from the fruit (preferably, the juice) using an extraction method that achieves the above-described particle size and particle size distribution can be used. The vesicular fraction may contain, for example, another component derived from the juice.

There is no particular limitation on the method for measuring the particle size of the vesicles; for example, nanoparticle tracking analysis and other methods can be adopted, and, for example, commercially available equipment (device names: NanoSight and qNANO) and the like can be used. The vesicles may be either extracellular vesicles or intracellular vesicles, for example, and extracellular vesicles are preferable. The vesicles may be, for example, exosome-like vesicles. The exosome-like vesicles are, for example, vesicles that are equal in size to human-derived extracellular vesicles (EVs), and more specifically, vesicles that are equal in size to small EVs (also referred to as exosomes) derived from human cells. The exosome-like vesicles are, for example, vesicles obtained using an isolation method similar to that for human cell-derived extracellular vesicles, and preferably a method similar to that for human cell-derived exosomes.

A "protein" is a compound in which amino acid are bonded together by peptide bonds. In the present invention, the length (number of amino acid residues) of a "protein" is not particularly limited, and the term "protein" in terms of length encompasses peptide (e.g., less than 50 residues), such as oligopeptide and polypeptide, and protein (e.g., 50 residues or more) with a length longer than that of peptide. The protein in the present invention may be, for example, an unmodified protein or a modified protein. There is no particular limitation on the size of the protein held by the protein carrier of the present invention, and the lower limit thereof is, for example, 10 kDa, 20 kDa, 30 kDa, 40 kDa, 50 kDa, or 80 kDa, and the upper limit is, for example, 150 kDa, 140 kDa, 130 kDa, 120 kDa, 110 kDa, 100 kDa, 90 kDa, 80 kDa, 70 kDa, 60 kDa, or 50 kDa, and the range thereof is, for example, 10 to 150 kDa, 20 to 150 kDa, 10 to 110 kDa, 20 to 110 kDa, 80 to 110 KDa, 50 to 80 KDa, 20 to 50 KDa, or the like.

A substance to be held by the protein carrier of the present invention may be, for example, a protein or a nucleic acid encoding the protein. The protein-coding nucleic acid is, for example, a ribonucleic acid, such as RNA or DNA. RNA is, for example, pre mRNA or mRNA, and is preferably mRNA. Further, the protein carrier of the present invention may hold the protein, may hold the protein-coding nucleic acid, may hold both the protein and the protein-coding nucleic acid, or may hold at least one of the protein and the protein-coding nucleic acid as well as other components. The other components can be selected as appropriate depending on, for example, the type of the substance to be held, the purpose of introducing the substance to be held into cells, and the like. When the other components are components that are selected depending on the type and/or purpose of the substance to be held, the other components will be referred to as "concomitant components" hereinafter.

There is no particular limitation on the type of the protein, which serves as the substance to be held, and the type thereof can be selected as appropriate depending on the purpose. Examples of the type of the protein include functional proteins such as enzymes, antibodies, antigen-binding fragments, and transcription factors. The size of the protein is not particularly limited, and examples thereof include the above-mentioned examples. The protein carrier of the present invention can be used, for example, in genetic engineering techniques such as genome editing, which will be described later. Thus, the protein may be, for example, an enzyme, specifically, a nuclease such as a sequence-specific nuclease.

Examples of the nuclease include Cas proteins such as Cas9 protein, ZFN (Zinc Finger Nuclease) proteins, and TALEN (Transcription Activator-Like Effector Nuclease) proteins.

The protein-coding nucleic acid, which serves as the substance to be protected, may be a nucleic acid constituted by a coding sequence for the protein, or may be a nucleic acid including a coding sequence for the protein. That is, the protein-coding nucleic acid may be any nucleic acid as long as it can express the protein of interest based on the coding sequence, for example, in cells into which the nucleic acid is introduced. The coding sequence in the protein-coding nucleic acid may be, for example, RNA such as mRNA, or DNA such as cDNA. The length of the coding sequence is not particularly limited, and can be, for example, the length calculated from the size or length of the protein. The size of the protein is, for example, as described above. Specific examples of the length of the coding sequence include a lower limit of, for example, 480 bases or 1200 bases, and an upper limit of, for example, 2640 bases, and a range of, for example, 480 to 2640 bases, 1200 to 2640 bases, 480 to 1200 bases, 1200 to 1920 bases, and 1920 to 2640 bases. The length of the protein-coding nucleic acid is not particularly limited, and may be, for example, the same as that of the coding sequence, or may be longer than the length of the coding sequence by about 1 to 300 bases, about 1 to 200 bases, or about 1 to 100 bases.

Examples of the protein-coding nucleic acid include a DNA molecule, an RNA molecule, and a chimeric molecule of DNA and RNA. The DNA molecule, the RNA molecule, and the chimeric molecule may be constituted by a natural nucleic acid, may be constituted by an artificial nucleic acid, or may be constituted by the natural nucleic acid and the artificial nucleic acid, for example.

The protein-coding nucleic acid may be, for example, an expression vector capable of expressing the protein, specifically, an expression vector in which a coding sequence for the protein is inserted into a vector such that the protein can be expressed. The coding sequence in the expression vector is, for example, DNA, preferably, cDNA. In the expression vector, for example, an antisense strand (template strand) against the amino acid sequence of the protein is inserted as DNA (e.g., cDNA), and specifically, a double-stranded DNA consisting of the antisense strand and a sense strand is inserted. The type of the vector encoding the amino acids of the protein is not particularly limited, and examples thereof include a plasmid vector and a viral vector. Examples of the viral vector include an adenovirus vector and a Sendai virus vector.

As described above, for example, the protein carrier of the present invention may hold at least one of the protein and the protein-coding nucleic acid, and further hold other components (the concomitant component).

The concomitant component is, for example, a nucleic acid substance (also referred to as a "concomitant nucleic acid component" hereinafter). Examples of the concomitant nucleic acid component include nucleic acid molecules such as a DNA molecule, an RNA molecule, and a chimeric molecule of DNA and RNA. The concomitant nucleic acid component may be constituted by a natural nucleic acid, may be constituted by an artificial nucleic acid, or may be constituted by the natural nucleic acid and the artificial nucleic acid, for example. The concomitant nucleic acid component may be, for example, an expression vector capable of expressing the nucleic acid molecule. When the nucleic acid molecule is a DNA molecule, for example, in the expression vector, an antisense strand (template strand) of DNA against the DNA molecule is inserted, and specifically, a double-stranded DNA consisting of the antisense strand and a sense strand is inserted. The type of the expression vector is not particularly limited, and examples thereof include a plasmid vector and a viral vector. Examples of the viral vector include lentiviral vectors and adenoviral vectors.

When the protein carrier of the present invention is used for the genome editing, examples of the concomitant nucleic acid component include a guide RNA (gRNA), and a specific example thereof is a single-stranded guide RNA (sgRNA). The concomitant nucleic acid component may be, for example, an expression vector capable of expressing the guide RNA (gRNA expression vector). Specifically, the gRNA expression vector may be, for example, an expression vector in which the guide RNA is inserted into the vector, or an expression vector in which the antisense strand (template strand) of DNA against the guide RNA, or a double-stranded DNA consisting of the antisense strand and the sense strand, is inserted into the vector. For example, a commercially available viral gRNA expression vector or the like can be used as the expression vector for the guide RNA. The guide RNA will be described later.

The protein carrier of the present invention may, for example, hold an expression vector for the protein and an expression vector for the concomitant nucleic acid component, which are independent of each other, or may hold an expression vector for the protein that co-expresses the protein and the nucleic acid molecule.

The protein carrier of the present invention may or may not contain the protein or the protein-coding nucleic acid, for example, at a stage prior to use by a user. When the protein carrier of the present invention contains the protein or the protein-coding nucleic acid, the protein carrier is also called a protein delivery reagent, as will be described below. When the protein carrier of the present invention contains the protein or the protein-coding nucleic acid, for example, it is preferable that the vesicles and the protein or the protein-coding nucleic acid form a conjugate (also referred to as a "vesicle conjugate" hereinafter). There is no particular limitation on the form of the vesicle conjugate, and, for example, the vesicle conjugate may have a form in which the protein or the protein-coding nucleic acid is encapsulated in the vesicles or a form in which the protein or the protein-coding nucleic acid is held by an outer wall (outer surface) of the vesicles. Specifically, for example, the vesicle conjugate has the latter form.

There is no particular limitation on the method for forming the vesicle conjugate. A method for forming a vesicle conjugate that holds the protein, and a method for forming a vesicle conjugate that holds the protein-coding nucleic acid will be described as examples. Note that the present invention is not limited to these examples.

The vesicle conjugate holding the protein can be formed by, for example, allowing the protein carrier of the present invention (i.e., the vesicles) and the protein to coexist in a solvent.

Specifically, for example, a conjugate of the vesicles and the protein can be formed by allowing the vesicles and the protein to coexist in a solvent and incubating them. There is no particular limitation on the incubation temperature, and, for example, the incubation temperature may be either within a room temperature range (e.g., 30°C ± 10°C) or a temperature range (e.g., above 0°C and below 20°C) lower than the room temperature range. Preferably, the incubation temperature is within a low temperature range (e.g., 4°C ± 5°C or 4°C ± 3°C), and the incubation is more preferably performed under ice cooling conditions (e.g., 1°C to 6°C).

In the coexistence, the ratio of the vesicles to the protein is not particularly limited, and is, for example, 5 × 10⁴ to 8 × 10⁴ vesicle particles, 2 × 10⁵ to 5 × 10⁵ vesicle particles, or 2 × 10⁶ to 5 × 10⁶ vesicle particles, with respect to 1 µmol of the protein. When the vesicles are caused to hold a Cas protein such as the Cas9 protein for use in the genome editing, the ratio of the vesicles to 1 µmol of the Cas protein is preferably, for example, in the above range. The same applies to, for example, nucleases other than the Cas proteins, and other proteins.

There is no particular limitation on the incubation time. The lower limit thereof is not particularly limited, and is, for example, 5 minutes or longer, or 15 minutes or longer. The upper limit is not particularly limited, and, for example, a plateau in conjugate formation can be reached through incubation for about 30 minutes. There is no particular limitation on the solvent. For example, an aqueous solvent can be used, and specific examples thereof include water, saline, and a buffer solution such as PBS.

In order to allow the vesicles to coexist with the protein, for example, a common introduction method such as electroporation or lipofection may be used. Note that, according to the protein carrier of the present invention, for example, the protein can be held by the vesicles simply by allowing the vesicles and the protein to coexist without using these common introduction methods.

Thus, according to the protein carrier of the present invention, for example, a conjugate of the vesicles and a protein of interest to be delivered to target cells can be formed simply by allowing the protein of interest to coexist with the vesicles in the solvent. Therefore, a substance (the protein) to be delivered by a DDS can be very easily held by the vesicles. Furthermore, according to the protein carrier of the present invention, for example, the protein held can be introduced into the target cells via the vesicles. Specifically, according to the protein carrier of the present invention, for example, the vesicles holding the protein fuse with the target cells, and the held protein is released into the target cells, thereby enabling introduction of the protein into the target cells from outside the target cells.

Note that in the case where the concomitant component is held by the vesicles together with the protein, it is sufficient to, for example, allow the vesicles, the protein, and the concomitant component to coexist in the solvent and incubate them. The conditions therefor are not particularly limited, and are similar to those described above as examples. In the case where the concomitant component is the concomitant nucleic acid component, incubation is preferably performed at a temperature within the above-mentioned low temperature range or lower, for example. This makes it possible to sufficiently prevent, for example, degradation of the concomitant nucleic acid component before the formation of the vesicle conjugate, due to the co-presence of a nucleic acid degrading enzyme such as RNase.

Next, the vesicle conjugate holding the nucleic acid encoding the protein can be formed in a similar manner as described above, for example, by allowing the protein carrier of the present invention (i.e., the vesicles) and the protein-coding nucleic acid to coexist in a solvent. Unless otherwise specified, the above-mentioned example of the method for forming the vesicle conjugate that holds the protein can be used.

Specifically, for example, a conjugate of the vesicles and the protein-coding nucleic acid can be formed by allowing the vesicles and the protein-coding nucleic acid to coexist in a solvent and incubating them. There is no particular limitation on the incubation temperature, and, for example, the incubation temperature may be either within a room temperature range (e.g., 30°C ± 10°C) or a temperature range lower than the room temperature range (e.g., above 0°C and below 20°C). Preferably, the incubation temperature is within a low temperature range (e.g., 4°C ± 5°C or 4°C ± 3°C, and the incubation is more preferably performed under ic e cooling conditions (e.g., 1°C to 6°C). In the case where the protein-coding nucleic acid is held in this manner, it is preferable to perform incubation at, for example, a temperature within the low temperature range or lower. This makes it possible to prevent, for example, degradation of the protein-coding nucleic acid before the formation of the vesicle conjugate, due to the co-presence of a nucleic acid degrading enzyme such as RNase.

In the coexistence, the ratio of the vesicles to the protein-coding nucleic acid is not particularly limited, and is, for example, 5 × 10⁴ to 8 × 10⁴ vesicle particles, 2 × 10⁵ to 5 × 10⁵ vesicle particles, or 2 × 10⁶ to 5 × 10⁶ vesicle particles, with respect to 1 µmol of the protein-coding nucleic acid. When the vesicles are caused to hold a Cas9-coding nucleic acid encoding the Cas9 protein for use in the genome editing, the ratio of the vesicles to 1 µmol of the Cas9-coding sequence is preferably in the above range. The coding sequence is, for example, Cas9 mRNA or Cas9 DNA encoding the Cas9 protein, and preferably Cas9 mRNA. Also, the protein-coding nucleic acid may be, for example, an expression vector into which the Cas9 mRNA or Cas9 DNA has been inserted. The same applies to, for example, nucleic acids encoding nucleases other than Cas proteins, and nucleic acids encoding other proteins.

There is no particular limitation on the incubation time. The lower limit thereof is not particularly limited, and is, for example, 5 minutes or longer, or 15 minutes or longer. The upper limit is not particularly limited, and, for example, a plateau in conjugate formation can be reached through incubation for about 30 minutes. There is no particular limitation on the solvent. For example, an aqueous solvent can be used, and specific examples thereof include water, saline, and a buffer solution such as PBS.

In order to allow the vesicles to coexist with the protein-coding nucleic acid, for example, a common introduction method such as electroporation or lipofection may be used. Note that, according to the protein carrier of the present invention, the protein-coding nucleic acid can be held by the vesicles without using these common introduction methods, for example, simply by allowing the vesicles and the protein-coding nucleic acid to coexist.

Thus, according to the protein carrier of the present invention, a conjugate of the vesicles and a nucleic acid encoding a protein of interest to be delivered to target cells can be formed, for example, simply by allowing the nucleic acid to coexist with the vesicles in the solvent. Therefore, a substance (the protein-coding nucleic acid) to be delivered by a DDS can be very easily held by the vesicles. Furthermore, according to the protein carrier of the present invention, for example, the protein-coding nucleic acid held thereby can be introduced into the target cells via the vesicles. Specifically, according to the protein carrier of the present invention, for example, the vesicles holding the protein-coding nucleic acid fuse with the target cells, and the protein-coding nucleic acid held thereby is released into the target cells, thereby enabling introduction of the protein-coding nucleic acid into the target cells from outside the target cells. Then, when the protein-coding nucleic acid is introduced into the target cells via the vesicles, for example, the protein encoded by the protein-coding nucleic acid will be expressed in the target cells, and as a result, the protein of interest will be introduced into the target cells via the vesicles.

Note that in the case where the concomitant component is also held by the vesicles together with the protein-coding nucleic acid, it is sufficient to, for example, allow the vesicles, the protein-coding nucleic acid, and the concomitant component to coexist in the solvent and incubate them. The conditions therefor are not particularly limited, and are similar to those described above as examples.

The protein or the protein-coding nucleic acid may be delivered by the protein carrier of the present invention, for example, in vitro, ex vivo, or in vivo.

A target to which the protein or the protein-coding nucleic acid is delivered by the protein carrier of the present invention may be, for example, a cell, or a tissue, an organ, or a living organism constituted by cells. The type of the target is not particularly limited, and examples thereof include a human and a non-human animal. Examples of the non-human animal include mice, rats, rabbits, dogs, monkeys, camels, and cows.

The protein carrier of the present invention is useful as, for example, pharmaceuticals, diagnostics, and agrochemicals, as well as research tools for agriculture, medicine, food science, life science, and the like.

### (2) Protein Delivery Reagent and Method for Producing the Same

The protein delivery reagent of the present invention includes the protein carrier of the present invention and the protein or the nucleic acid encoding the protein. The protein delivery reagent of the present invention includes the above-described vesicles of a fruit of a plant in the family Malpighiaceae as the carrier for a protein or the protein-coding nucleic acid, and there are no particular limitations on the other configurations and conditions.

In the protein delivery reagent of the present invention, the vesicles hold the protein or the protein-coding nucleic acid. That is, the protein delivery reagent of the present invention contains, for example, a conjugate of the vesicles and the protein or the protein-coding nucleic acid (the vesicle conjugate). The description of the protein carrier of the present invention can be applied to the protein delivery reagent of the present invention.

Also, a method for producing a protein delivery reagent according to the present invention includes allowing the protein carrier of the present invention (carrier including vesicles of the fruit of the plant in the family Malpighiaceae) and the protein to be delivered or the nucleic acid encoding the protein to coexist in a solvent to thereby form a conjugate of the vesicles of the carrier for a protein and the protein or the nucleic acid encoding the protein. The feature of the production method of the present invention is that the vesicles are used as carriers, and there are no particular limitations on the other configurations and conditions.

The description of the method for forming the vesicle conjugate in the protein carrier of the present invention can be applied to the method for producing a protein delivery reagent of the present invention.

### (3) Protein Introduction Method

As described above, the method for introducing a protein into cells according to the present invention includes bringing a conjugate (the vesicle conjugate) of the protein carrier of the present invention and a protein to be delivered or a nucleic acid encoding the protein into contact with cells. The feature of the protein introduction method according to the present invention is that the above-described vesicles of the fruit of the plant in the family Malpighiaceae, i.e., the protein carrier of the present invention, are used as carriers for delivery, and there are no particular limitations on the other configurations and conditions. Also, in the protein introduction method according to the present invention, the descriptions in the sections (1) and (2) above can be applied to, for example, the protein carrier, the protein delivery reagent in which the protein carrier holds the protein or the protein-coding nucleic acid, the production methods therefor, and the like, unless otherwise specified.

In the protein introduction method according to the present invention, the vesicle conjugate may be a conjugate containing the concomitant component in addition to the protein or the protein-coding nucleic acid.

In the protein introduction method according to the present invention, for example, the vesicle conjugate (the protein delivery reagent of the present invention) prepared in advance may be used, or the protein introduction method may further include forming the vesicle conjugate. That is, the protein introduction method according to the present invention may include forming a vesicle conjugate of the protein carrier and the protein or the nucleic acid encoding the protein, prior to the bringing of the conjugate into contact with the cells.

Further, in the protein introduction method according to the present invention, in the forming, for example, a conjugate of the protein carrier, the protein or the nucleic acid encoding the protein, and the concomitant component may be formed as the vesicle conjugate.

In the protein introduction method according to the present invention, the bringing of the conjugate into contact with the cells is, for example, bringing the vesicle conjugate into contact with cells in vivo, ex vivo, or in vitro.

There is no particular limitation on a target to which the vesicle conjugate is administered, and examples thereof include cells, tissues, organs, and living organisms, and there is no particular limitation on the type thereof. In the case where the administration target is a living organism, the bringing of the conjugate into contact with the cells is, for example, administering the vesicle conjugate to a human or a non-human animal. Examples of the non-human animal include mice, rats, rabbits, dogs, monkeys, camels, and cows.

In the case where the vesicle conjugate is administered in vivo in the bringing of the conjugate into contact with the cells, there is no particular limitation on the route of administration, and examples thereof include oral administration and parenteral administration. Examples of the parenteral administration include intravenous administration, intra-arterial administration, intramuscular administration, subcutaneous administration, intraperitoneal administration, topical administration, and the like.

According to the present invention, since the protein carrier of the present invention including the vesicles is used, the substance that is to be delivered by a DDS and is held by the vesicle conjugate can be introduced into the target cells simply by bringing the vesicle conjugate into contact with the target cells without using common introduction methods such as electroporation, a transfection reagent, and microinjection. Further, as described above, the protein carrier of the present invention is derived from a plant and does not require a transfection reagent or the like. Therefore, an influence on the cells, living organisms, or the like into which the protein is introduced can also be suppressed. The protein carrier of the present invention is also effective for oral administration, for example, since the protein carrier is hardly affected by gastric acid.

### [Embodiment 1]

The protein carrier of the present invention is capable of delivering, for example, a protein to target cells, and can therefore be used, for example, as a tool for genome editing, as described above.

In the case where the protein carrier of the present invention is used for genome editing, it is preferable that the protein carrier of the present invention holds, for example, a nuclease that can be used for genome editing. Examples of the nuclease include Cas proteins such as Cas9 protein, ZFN proteins, and TALEN proteins.

The following describes an example in which the protein carrier of the present invention holds the Cas9 protein, which is a Cas protein, and is used for genome editing, or an example in which the protein carrier holds a Cas9 expression vector, in which the coding sequence thereof has been inserted into a vector (e.g., a plasmid vector), and is used for genome editing. Note that the present invention is not limited to these examples, and for example, Cas9 can be replaced with other nucleases of the Cas protein family, and other nucleases.

### (1) Cas9 Protein

An example of a genome editing system that uses the Cas9 protein is the so-called CRISPR-Cas9 system. In this system, an RNP complex (ribonucleotideprotein) (Cas9/gRNA) is used to cleave a sequence of interest in the genome inside the cell. The RNP complex is a complex of the Cas9 protein and a guide RNA (gRNA). A gRNA is a sequence that is designed to specifically recognize, i.e., bind specifically to, a target sequence of a gene of interest in the genome. Regarding gRNAs, a single-stranded gRNA (also referred to as "sgRNA" hereinafter) is usually used for forming the RNP. When the RNP complex is introduced into target cells, the gRNA of the RNP complex specifically recognizes the target sequence, and the Cas9 protein of the RNP complex cleaves the target sequence. This cleavage allows, for example, knock-out of a gene of interest in the genome. Knock-in is also possible by inserting a foreign gene by utilizing the cell's repair mechanism associated with cleavage. In the latter case, for example, a donor DNA is allowed to coexist as the foreign gene.

### (1-1) Cas9 Protein + gRNA

In the case where the protein carrier of the present invention is used in the CRISPR-Cas9 system, for example, it is sufficient that the vesicles, Cas9 protein, and gRNA are allowed to coexist in the solvent and incubated. There are no particular limitations on the incubation conditions, and examples described above can be applied. This allows the vesicles to hold the CAS9 protein and the gRNA and form the vesicle conjugate. Furthermore, by adding the vesicle conjugate to target cells of interest, the vesicle conjugate fuses with the target cells, and the RNP complex of the CAS9 protein and the gRNA that is held by the vesicles is introduced into the target cells.

The Cas9 protein and the gRNA may, for example, be brought into contact with each other in advance to form the RNP, and then the RNP may be allowed to coexist with the vesicles in the solvent, or the vesicles, the Cas9 protein, and the gRNA may be separately added to the solvent without being brought into contact with each other in advance and then allowed to coexist with each other. Also, in the solvent, the Cas9 protein may be brought into contact with the vesicles, and then the gRNA may be brought into contact with the vesicles. The Cas9 protein and the gRNA can form the RNP, for example, through contact with each other. The vesicles (the vesicle conjugate) that hold the RNP can be formed in this manner.

There is no particular limitation on the method for introducing the vesicle conjugate holding the RNP into the target cells, and, for example, it is sufficient that the vesicle conjugate is allowed to coexist with the target cells. As a result of the vesicle conjugate being allowed to coexist with the target cells, for example, the vesicles of the vesicle conjugate fuse with cell membranes of the target cells, and the RNP is released from the vesicle conjugate into the cells. The RNP released into the cells is transferred to the nuclei of the target cells, acts on the target sequence, and then is degraded in the cells.

### (1-2) Cas9 Protein + Oligonucleotides against gRNA

The vesicles may hold, for example, an oligonucleotide against the gRNA (e.g., an antisense strand of DNA), instead of the gRNA. In the case of the oligonucleotide, a vesicle conjugate may be formed by bringing the oligonucleotide into contact with the vesicles together with the Cas9 protein and thereby causing the vesicles to hold them. By adding the vesicle conjugate to the target cells, the vesicle conjugate fuses with the target cells, and the Cas9 protein and the oligonucleotide are introduced into the target cells. Then, in the cytoplasm of the target cells, the gRNA is expressed from the oligonucleotide, and a RNP complex of the Cas9 protein and the gRNA is formed.

### (1-3) Cas9 Protein + gRNA Expression Vector

The vesicles may hold, for example, the gRNA expression vector, instead of the gRNA. In the case of the gRNA expression vector, a vesicle conjugate may be formed by bringing the gRNA expression vector into contact with the vesicles together with the Cas9 protein and thereby causing the vesicles to hold them. By adding the vesicle conjugate to the target cells, the vesicle conjugate fuses with the target cells, and the Cas9 protein and the gRNA expression vector are introduced into the target cells. Then, in the cytoplasm of the target cells, the gRNAis expressed, and a RNP complex of the Cas9 protein and the gRNA is formed. The RNP is transferred to the nuclei of the target cells, acts on the target sequence, and then is degraded in the cells.

### (2) Cas9 Protein Coding Sequence

In the case where a coding sequence (e.g., Cas9 mRNA) is used as a Cas9 protein coding nucleic acid, for example, it is sufficient that the vesicles, the Cas9 mRNA, and the gRNA are allowed to coexist in the solvent, and incubated. There are no particular limitations on the incubation conditions, and examples described above can be applied. This allows the vesicles to hold the Cas9 mRNA and the gRNA and form the vesicle conjugate. Furthermore, by adding the vesicle conjugate to target cells of interest, the vesicle conjugate fuses with the target cells, and the Cas9 mRNA and the gRNA that are held by the vesicles are introduced into the target cells. The Cas9 mRNA introduced into the target cells expresses the Cas9 protein in the cytoplasm of the target cells, and forms a RNP complex with the co-introduced gRNA. The RNP is then transferred to the nuclei of the target cells, acts on the target sequence, and then is degraded in the cells.

The examples described in (1) above can be applied to the gRNA. That is, for example, the gRNA may be directly held by the vesicles as shown in (1-1) above, an oligonucleotide against the gRNA may be held by the vesicles as shown in (1-2) above, or the gRNA expression vector may be held by the vesicles as shown in (1-3) above.

In the case of the gRNA, a vesicle conjugate may be formed by, for example, bringing the gRNA into contact with the vesicles together with the Cas9 mRNA and thereby causing the vesicles to hold them. By adding the vesicle conjugate to the target cells, the vesicle conjugate fuses with the target cells, and the Cas9 mRNA and the gRNA are introduced into the target cells. Then, in the cytoplasm of the target cells, the Cas9 protein is expressed, and a RNP complex of the Cas9 protein and the gRNA is formed. The RNP is transferred to the nuclei of the target cells, acts on the target sequence, and then is degraded in the cells.

Also, in the case of the oligonucleotide, a vesicle conjugate may be formed by bringing the oligonucleotide into contact with the vesicles together with the Cas9 mRNA and thereby causing the vesicles to hold them. By adding the vesicle conjugate to the target cells, the vesicle conjugate fuses with the target cells, and the Cas9 mRNA and the oligonucleotide are introduced into the target cells. Then, in the cytoplasm of the target cells, the Cas9 protein and the gRNA are expressed, and a RNP complex is formed. The RNP is transferred to the nuclei of the target cells, acts on the target sequence, and then is degraded in the cells.

In the case of the gRNA expression vector, a vesicle conjugate may be formed by bringing the gRNA expression vector into contact with the vesicles together with the Cas9 mRNA and thereby causing the vesicles to hold them. By adding the vesicle conjugate to the target cells, the vesicle conjugate fuses with the target cells, and the Cas9 mRNA and the gRNA expression vector are introduced into the target cells. Then, in the cytoplasm of the target cells, the Cas9 protein and the gRNA are expressed, and a RNP complex is formed. The RNP is transferred to the nuclei of the target cells, acts on the target sequence, and then is degraded in the cells.

### (3) Cas9 Expression Vector

In the case where the Cas9 expression vector is used, for example, it is sufficient that the vesicles, the Cas9 expression vector, and the gRNA are allowed to coexist in the solvent and incubated. There are no particular limitations on the incubation conditions, and examples described above can be applied. This allows the vesicles to hold the Cas9 expression vector and the gRNA and form the vesicle conjugate. Furthermore, by adding the vesicle conjugate to target cells of interest, the vesicle conjugate fuses with the target cells, and the Cas9 expression vector and the gRNA that are held by the vesicles are introduced into the target cells. The Cas9 expression vector introduced into the target cells expresses the Cas9 protein in the cytoplasm of the target cells, and forms an RNP complex with the co-introduced gRNA. The RNP is then transferred to the nuclei of the target cells, acts on the target sequence, and then is degraded in the cells.

The examples described in (1) above can be applied to the gRNA. That is, for example, the gRNA may be directly held by the vesicles as shown in (1-1) above, an oligonucleotide against the gRNA may be held by the vesicles as shown in (1-2) above, or the gRNA expression vector may be held by the vesicles as shown in (1-3) above.

In the case of the gRNA, a vesicle conjugate may be formed by, for example, bringing the gRNA into contact with the vesicles together with the Cas9 expression vector and thereby causing the vesicles to hold them. By adding the vesicle conjugate to the target cells, the vesicle conjugate fuses with the target cells, and the Cas9 expression vector and the gRNA are introduced into the target cells. Then, in the cytoplasm of the target cells, the Cas9 protein is expressed, and an RNP complex of the Cas9 protein and the gRNA is formed. The RNP is transferred to the nuclei of the target cells, acts on the target sequence, and then is degraded in the cells.

Also, in the case of the oligonucleotide, a vesicle conjugate may be formed by bringing the oligonucleotide into contact with the vesicles together with the Cas9 expression vector and thereby causing the vesicles to hold them. By adding the vesicle conjugate to the target cells, the vesicle conjugate fuses with the target cells, and the Cas9 expression vector and the oligonucleotide are introduced into the target cells. Then, in the cytoplasm of the target cells, the Cas9 protein and the gRNA are expressed, and an RNP complex is formed. The RNP is transferred to the nuclei of the target cells, acts on the target sequence, and then is degraded in the cells.

In the case of the gRNA expression vector, a vesicle conjugate may be formed by bringing the gRNA expression vector into contact with the vesicles together with the Cas9 expression vector and thereby causing the vesicles to hold them. By adding the vesicle conjugate to the target cells, the vesicle conjugate fuses with the target cells, and the Cas9 expression vector and the gRNA expression vector are introduced into the target cells. Then, in the cytoplasm of the target cells, the Cas9 protein and the gRNA are expressed, and an RNP complex is formed. The RNP is transferred to the nuclei of the target cells, and the Cas9 protein of the RNP is degraded in the cells.

### [Embodiment 2]

As shown in the Embodiment 1 above, the protein carrier of the present invention makes it possible to easily introduce a protein such as a Cas protein or a protein-coding nucleic acid into target cells. Thus, the present invention can be used to treat various diseases using genome editing or the like. There are no particular limitations on the type of disease, the type of target cells, and the type of target gene, and examples of the use of the present invention include the following diseases. Note that these are merely examples and do not limit the present invention.
(1) Treatment target: Transthyretin amyloidosis
   Target organ: Liver
(2) Hypercholesterolemia
   Target gene: PCSK9

Hereinafter, the present invention will be described in greater detail using examples and the like. However, the present invention is not limited to the examples and the like below.

### Examples

### [Working Example 1]

The function of acerola-derived vesicles as a protein carrier was confirmed through genome editing. Kits, reagents, apparatuses, and the like were used according to their instruction manuals. Unless otherwise specified, the conditions for each process were the same in the following working examples.

### (Working Example 1-1)

The genome editing was carried out in vitro.

### (1) Preparation of Vesicles

First, 10 mL of juice from fully ripe acerola fruit from Okinawa was concentrated 10-fold (1 mL) using an ultrafiltration filter (trade name: Amicon (trademark) Ultra-15 Centrifugal Filter Units-50,000 NMWL, available from Millipore) with a nominal molecular weight limit (NMWL) of 50,000. Then, 1 mL of the resulting concentrate was ultracentrifuged (4°C, 4 hours, 145,000 ×g) to collect a precipitated fraction containing vesicles. The precipitated fraction was suspended in 120 µL of PBS to obtain a vesicle sample A. The vesicle sample A obtained from 10 mL of the juice was subjected to a nanoparticle analysis system (trade name: NanoSight, available from Malvern), and a particle size distribution of vesicles contained in the vesicular fraction was checked. The results are shown below.
Particle amount: 1.9 × 10⁹ particles/pL
Particle size:
   Mean 248 nm
   Mode 138 nm
   SD 128 nm

Using 10 mL of the same juice as that used to prepare the sample A, a vesicle sample B containing vesicles was prepared using a commercially available kit (trade name: Qiagen exoEasy kit, available from Qiagen). Specifically, treatment was carried out as follows. First, 10 mL of the juice was filtered using a membrane filter (trade name: Durapore (trademark) PVDF Membrane Filter, available from Millipore) with a pore size of 0.45 µm. Then, 10 mL of the obtained filtrate was applied to the kit, and an eluted fraction containing vesicles was separated (isolated) through elution using 400 µL of the Buffer (Buffer XE) of the kit. Then, the eluted fraction was subjected to ultracentrifugation (100,000 ×g, 49000 rpm, 70 minutes, 4°C) to collect vesicle pellets. The vesicle pellets were suspended in 50 µL of PBS to obtain a vesicle sample B. Also, the vesicle sample B obtained from 10 mL of the juice was subjected to a nanoparticle analysis system (trade name: NanoSight, available from Malvern), and a particle size distribution of vesicles contained in the vesicle fraction was checked. The results are shown below.
Particle amount: 2.2 × 10⁸ particles/pL
Particle size:
   Mean 208 nm
   Mode 155 nm
   SD 108 nm

### (2) RNP Formation

A synthetic gRNA against human alpha-galactosidase (GLA) was used as a concomitant component, and a Cas9 recombinant protein (trade name: Alt-R S.p. Cas9 Nuclease V3, available from IDT) was used as a Cas9 protein. The synthetic gRNA was prepared using a commercially available reagent (trade name: Alt-R CRISPR-Cas9 crRNA, tracrRNA, available from IDT) according to the instructions. Specifically, the synthetic gRNA was prepared by mixing the crRNA that recognizes the GLA with the tracRNA that binds to the CAS9 protein, and heating (95°C) and then annealing the mixture at room temperature for 15 minutes. The sequence of the Cas9 protein is registered in the database (GenBank) under Accession No. CDJ55032. 15 µL of the gRNA (1 µmol/L) and 15 µL of the Cas9 protein (1 µmol/L) were mixed, and the reaction of the resulting mixture was allowed to proceed at room temperature for 15 minutes to generate an RNP. The mixture after the reaction was used as an RNP sample.

### (3) Formation of Vesicle Conjugate

20 µL of the vesicle sample (A or B) and 30 µL of the RNP sample were mixed in a microtube, and the reaction of the resulting mixture was allowed to proceed on ice for 30 minutes. The entire amount of the mixture after the reaction was used as an RNP-vesicle conjugate sample. An RNP-vesicle conjugate sample A was prepared using the vesicle sample A, and an RNP-vesicle conjugate sample B was prepared using the vesicle sample B.

### (4) Introduction into Cells

Using a DMEM/F12 (Gibco) medium containing 10% FBS, human embryonic kidney cells HEK293 were seeded on a 96-well plate (1 × 10⁴ cells/well) and cultured at 37°C. After 24 hours of the culture, 50 µL of the RNP-vesicle conjugate sample (A or B) was added to each of the wells, and the cells were further cultured. After 48 hours of the culture from the addition of the RNP-vesicle conjugate sample, genomic DNA was extracted from the cultured cells in the wells. Then, for the obtained DNA extraction sample, a region (500 bp) containing the PAM sequence of the GLA and the target sequence of the gRNA was amplified by PCR, and the sequence was analyzed through direct sequencing by means of Sanger sequencing.

For the negative control (NC), cells were cultured in the same manner, except that the vesicle sample was not used, and 30 µL of the RNP sample was added to the cells instead of the RNA-vesicle conjugate sample, and the sequence was analyzed.

For the positive control (PC), the RNP was introduced into the cells using a commercially available transfection reagent (trade name: Lipofectamin RNAiMAX, available from Thermo Fisher Scientific), instead of the vesicle sample. Specifically, the cells were cultured in the same manner, except that 30 µL of the RNP sample and 1.2 µL of the transfection reagent were mixed in a microtube and the reaction was allowed to proceed at room temperature for 20 minutes, and the entire amount of the mixture after the reaction was added to the cells, and the sequence was analyzed.

The waveform data from the sequence analysis was analyzed using the web tool (http://shinyapps.datacurators.nl/tide/) of the TIDE (Tracking of Indels by Decomposition) to calculate the INDEL rate (INDEL insertion rate) in the sequence of the amplified region. The results are shown in the table below.

**[Table 1]**

| | Vesicle Sample | Lipofectamine | TIDE Total eff. (%) |
|---|---|---|---|
| Work. Ex. | A | - | 7.0 |
| | B | - | 14.2 |
| PC | - | + | 23.8 |
| NC | - | - | 0.0 |

As shown in Table 1, in the NC case, in which neither the vesicle sample nor Lipofectamine was used, when the RNP was simply brought into contact with the cells, no INDELs (insertion and/ deletion of bases) were observed downstream of the PAM sequence in the GLA gene (0%). In the PC case, in which Lipofectamine was used, it was confirmed that the RNP was introduced into the cells by Lipofectamine, and a double-strand break in the genomic DNA at the PAM sequence of the GLA gene and insertion of a sequence during genomic DNA repair caused by the double-strand break were confirmed. In contrast, in the working examples, in which vesicle samples derived from an acerola were used, like the PC case, double-strand breaks and insertions were confirmed in all cases where the vesicle samples were used. Based on this, it was confirmed that, with the vesicle samples, an RNP-vesicle conjugate that holds the RNP was formed simply by bringing the vesicle sample into contact with the RNP, and the RNP was introduced into the cells simply by bringing the RNP-vesicle conjugate into contact with the cells, and the introduced RNP functioned in the cells.

### (Working Example 1-2)

The genome editing was carried out in vivo.

The vesicle sample A of Working Example 1-1 was used as the vesicle sample. The concentration of the vesicle sample was adjusted using PBS. Then, a Cas9/gRNA complex (RNP) of the Cas recombinant protein (trade name: Alt-R S.p. Cas9 Nuclease V3, available from IDT) and a synthetic gRNA against the human alpha-galactosidase (GLA) was produced in the same manner as in Working Example 1-1.

C57BL/6 mice were used (n = 3). 0.05 mL of the vesicle sample A (1 × 10¹¹ particles/mL) and 0.05 mL of the RNP (100 nmol/L) were mixed, and the resulting mixture was incubated on ice for 30 minutes to form a conjugate. Then, 100 µL of the mixture after the reaction was used as an RNP-vesicle conjugate sample. PBS was added to 100 µL of the RNP-vesicle conjugate sample to make up a total volume of 200 µL, and the resulting mixture was orally administered to each mouse using a gastric tube. Twenty-four hours after oral administration, the small intestine and the liver were collected from the mouse, and DNA was extracted from each tissue using a DNEASY^{®} Blood&Tissue kit (Qiagen). For the DNA extraction sample of each tissue, a region containing the PAM sequence of the GLA and the target sequence of the gRNA was amplified by PCR, and the sequence was analyzed through direct sequencing by means of Sanger sequencing, in the same manner as in Working Example 1-1.

As for the negative control, cells were cultured in the same manner, except that the vesicle sample was not used, and PBS was added to 0.05 mL of RNP (100 nmol/L) to make up a total volume of 200 µL, and the thus prepared mixture was then administered to each mouse instead of the RNA-vesicle conjugate sample, and the sequence was analyzed.

Then, the INDEL rate in the sequence of the amplified region was calculated using the web tool of the TIDE in the same manner as in Working Example 1-1. The results are shown in the tables below.

**[Table 2]**

| (Small Intestine) | | |
|---|---|---|
| | Vesicle Sample | TIDE Total eff. (%) |
| Work. Ex. | A | 14.8% |
| NC | - | 0.1% |

| (Liver) | | |
|---|---|---|
| | Vesicle Sample | TIDE Total eff. (%) |
| Work. Ex. | A | 8.6% |
| NC | - | 0% |

As shown in Table 2, in the NC case, in which neither the vesicle sample nor Lipofectamine was used, no INDELs (insertion and/ deletion of bases) were observed downstream of the PAM sequence in the GLA gene in either the small intestine or liver when the RNP was administered alone (0.1%, 0%). In contrast, in the working examples, in which vesicle samples derived from an acerola were used, double-strand breaks and insertions were confirmed in both the small intestine and liver. Based on this, it was confirmed that, with the vesicle sample, an RNP-vesicle conjugate holding the RNP was formed simply by bringing the vesicle sample into contact with the RNP, and the RNP was introduced into the cells in living organisms simply by administering the RNP-vesicle conjugate, and the introduced RNP functioned in the cells.

As described above, according to the protein carrier of the present invention, the protein and the concomitant component (nucleic acid substance) can be held by vesicles simply by bringing the protein and the concomitant component into contact with the vesicles, without using a transfection reagent such as Lipofectamine. Furthermore, the protein carrier can, for example, introduce the protein and the concomitant component, which are held by the protein carrier, into cells, regardless of whether genome editing is in vivo or in vitro. Therefore, it can be said that the protein carrier will be a useful delivery tool for genome editing mentioned in this working example as an example, as well as other applications for introducing a protein into cells.

### [Working Example 2]

In this working example, instead of the Cas9 protein in Working Example 1 above, Cas9 mRNA (database GenBank, accession No. CDJ55032) encoding the ORF of the Cas9 protein was used to confirm the function of the protein vesicles through genome editing.

The vesicle sample B of Working Example 1 was used as the vesicle sample. Then, instead of the Cas9 protein, 500 ng of the Cas9 mRNA having 4104 bases, and 15 µL of the gRNA (1 µmol/L), which was the same as in Working Example 1 above, were mixed with 20 µL of the vesicle sample, and the reaction was allowed to proceed on ice for 30 minutes. The entire amount of the mixture after the reaction was used as a vesicle conjugate sample. Addition of the vesicle conjugate sample to the cells (HEK293), culture, extraction of genomic DNA, sequence analysis, and calculation of the INDEL rate were conducted in the same manner as in the Working Example 1 above, except for using the vesicle conjugate sample. The results are shown in the table below.

**[Table 3]**

| | Vesicle Sample | Lipofectamine | TIDE Total eff. (%) |
|---|---|---|---|
| Work. Ex. | B | - | 12.4 |
| PC | - | + | 15.4 |
| NC | - | - | 0.2 |

As shown in Table 3, in the NC case, in which neither the vesicle sample nor Lipofectamine was used, virtually no INDELs (insertion and/or deletion of bases) were observed downstream of the PAM sequence in the GLA gene (0.2%). In the PC case, in which Lipofectamine was used, a double-strand break in the genomic DNA at the PAM sequence of the GLA gene and insertion of a sequence during genomic DNA repair following the double-strand break were confirmed. In contrast, in the working example, in which the vesicle sample derived from an acerola was used, a double-strand break and an insertion were confirmed, like the PC case. Based on this, it was confirmed that, with the vesicle sample, a vesicle conjugate holding the mRNA and the gRNA was formed simply by bringing the vesicle sample into contact with the RNP, the mRNA and the gRNA were introduced into cells simply by bringing the cells into contact with the vesicle conjugate, and the introduced mRNA was translated into the Cas9 protein in the cells, the Cas9 protein and the gRNA formed the RNP, and the RNP functioned in the cells.

As described above, according to the protein carrier of the present invention, a nucleic acid encoding a protein and a concomitant component (nucleic acid substance) can be held by vesicles simply by bringing the nucleic acid encoding the protein and the concomitant component into contact with the protein carrier, without using a transfection reagent such as Lipofectamine, and the nucleic acid encoding the protein and the concomitant component can be introduced into the cells. Therefore, it can be said that the protein carrier will be a useful delivery tool for genome editing mentioned in this working example as an example, as well as other applications for introducing a protein into cells.

### [Embodiment 3]

It was confirmed that the vesicles derived from the acerola can be used as protein carriers for proteins other than the Cas9 protein.

The vesicle sample B of Working Example 1-1 was used as the vesicle sample. 0.01 mL of the vesicle sample (2 × 10⁷ particles/mL) whose concentration had been adjusted with PBS was mixed with 0.005 mL of anti-mTOR antibody (mouse IgG, Cell Signaling Technology, 2 µg) against human mTOR, and the vesicle sample and the anti-mTOR antibody were incubated on ice for 30 minutes to form a conjugate thereof. The mixture after the reaction was used as an antibody-vesicle conjugate sample.

Using a DMEM/F12 medium containing 10% FCS (fetal calf serum), SiHa cells (a human cervical cancer cell line) were seeded on a 6-well plate and cultured at 37°C until they reached 50% confluence. Then, 10 µL of the antibody-vesicle conjugate sample (final concentration of 1 × 10⁵ particles/mL, based on the number of acerola vesicle particles) was added to each well, and the cells were cultured for 24 hours. After 24 hours of the culture, the number of cells in each well was measured using a measuring device (trade name: Cell Titer-Glo, available from Promega).

In addition, cells were also cultured in the same manner in the following systems: as a control 1, in a system in which no antibody-vesicle conjugate sample was added; as a control 2, in a system in which only the vesicle sample was added (2 × 10⁷ particles/mL, 0.01 mL); and as a control 3, in a system in which only anti-mTOR antibody (2 µg) was added. Then, the number of cells in each system was measured in the same manner. The number of cells in the control 1 was set as a relative value of 1, and the relative value of the number of cells was calculated as the cell viability.

Figure 1 shows the results. Figure 1 is a graph showing the cell viability, with the cell viability being shown on the vertical axis. As shown in Figure 1, the controls 1, 2, and 3 showed approximately similar viabilities. In contrast, in the working examples, in which the antibody-vesicle conjugate sample was added, a decrease in the number of cells, i.e., a decrease in the viability, was observed, compared to the controls. The anti-mTOR antibody is known to reduce the cell viability by neutralizing the function of mTOR, which is involved in the PI3K/Akt/mTOR signal pathway. Therefore, the above results supported that the anti-mTOR antibody was delivered to the cells by the acerola vesicles and that mTOR functioned in the cells.

As described above, it was revealed that the protein carrier of the present invention can hold and introduce into cells not only the Cas9 protein as in Working Examples 1 and 2 above, but also various proteins, including antibodies as in this working example.

Although the invention of the present application has been described above with reference to the embodiments, the invention is not limited to the above embodiments. Various changes that may be understood by those skilled in the art can be made to the configuration and details of the invention of the present application without departing from the scope of the invention.

This application claims the benefit of priority from Japanese Patent Application No. 2022-156126, filed on September 29, 2022, the entire contents of which are incorporated herein by reference.

### Industrial Applicability

The carrier for a protein according to the present invention includes vesicles of a fruit of a plant in the family Malpighiaceae, such as acerolas, and can hold a protein or a nucleic acid encoding the protein by the vesicles. Therefore, the carrier for a protein according to the present invention can be used as a DDS tool for proteins, such as nucleases used in, for example, genome editing or the like.

## Claims

1. A carrier for a protein, comprising
vesicles of a fruit of a plant in the family Malpighiaceae,
wherein a substance to be held is a protein or a nucleic acid encoding the protein.

2. The carrier for a protein according to claim 1,
wherein the plant in the family Malpighiaceae is an acerola plant species.

3. The carrier for a protein according to claim 1 or 2,
wherein the vesicles have an average particle size of 30 to 400 nm.

4. The carrier for a protein according to any one of claims 1 to 3,
wherein the vesicles are extracellular vesicles.

5. The carrier for a protein according to any one of claims 1 to 4,
wherein the nucleic acid encoding the protein is an expression vector in which a coding sequence for the protein is inserted into a vector.

6. The carrier for a protein according to any one of claims 1 to 5,
wherein the protein is a sequence-specific nuclease.

7. The carrier for a protein according to claim 6,
wherein the sequence-specific nuclease is a Cas protein.

8. The carrier for a protein according to claim 7,
wherein the Cas protein is a Cas9 protein.

9. The carrier for a protein according to any one of claims 1 to 8,
wherein the substance to be held further contains a concomitant component for the protein or the nucleic acid encoding the protein.

10. The carrier for a protein according to claim 9,
wherein the concomitant component is a nucleic acid substance, and the nucleic acid substance is a guide RNA.

11. The carrier for a protein according to any one of claims 1 to 10, further comprising
the protein or the nucleic acid encoding the protein.

12. A method for producing a protein delivery reagent, comprising
allowing the carrier for a protein including the vesicles according to any one of claims 1 to 10 and a protein to be delivered or a nucleic acid encoding the protein to coexist in a solvent to thereby form a conjugate of the vesicles of the carrier for a protein and the protein or the nucleic acid encoding the protein.

13. The method for producing a protein delivery reagent according to claim 12, further comprising
mixing and incubating the carrier for a protein and the protein or the nucleic acid encoding the protein in the solvent.

14. The method for producing a protein delivery reagent according to claim 12 or 13, further comprising
allowing a concomitant component for the protein or the nucleic acid encoding the protein to coexist in the solvent together with the carrier for a protein.

15. A method for introducing a protein into cells, comprising
bringing a conjugate of the carrier for a protein including the vesicles according to any one of claims 1 to 10 and a protein to be delivered or a nucleic acid encoding the protein into contact with cells.

16. The protein introduction method according to claim 15,
wherein the conjugate is a conjugate further containing a concomitant component for the protein or the nucleic acid encoding the protein.

17. The protein introduction method according to claim 15 or 16, further comprising
prior to the bringing of the conjugate into contact with the cells,
forming a conjugate of the carrier for a protein and the protein or the nucleic acid encoding the protein.

18. The protein introduction method according to any one of claims 15 to 17,
wherein the bringing of the conjugate into contact with the cells is bringing the conjugate into contact with cells in vivo, ex vivo, or in vitro.

19. The protein introduction method according to any one of claims 15 to 18,
wherein the bringing of the conjugate into contact with the cells is administering the conjugate to a human or a non-human animal.
